# EUROPÄISCHE PATENTSCHRIFT

(11) **EP 1 719 753 B1**
(45) Veröffentlichungstag und Bekanntmachung des Hinweises auf die Patenterteilung: **19.05.2010**
(21) Anmeldenummer: 06111045.8
(22) Anmeldetag: 13.03.2006
(51) Int. Cl.: C07C 67/08, C07C 67/48, C07C 69/75, C07C 69/76, C07C 69/78, C07C 69/80

(54) **Verfahren zur Herstellung von Carbonsäureestern**
Process for the preparation of carboxylic acid esters
Procédé pour la préparation des esters d'acides carboxyliques

(30) Priorität: 06.05.2005 DE 102005021075
(43) Veröffentlichungstag der Anmeldung: 08.11.2006
(73) Patentinhaber: Evonik Oxeno GmbH, 45772 Marl (DE)
(72) Erfinder: Gubisch, Dr. Dietmar, 45772, Marl (DE); Beck, Thomas, 44139, Dortmund (DE); Büschken, Dr. Wilfried, 45721, Haltern am See (DE)
(74) Vertreter: Hirsch, Hans-Ludwig

(56) Entgegenhaltungen:
- EP-A- 1 300 388
- EP-A- 1 354 867
- WO-A-2005/037764
- DE-A1- 2 112 492
- DE-A1- 2 320 641
- DE-A1- 2 330 435
- FR-A- 1 520 215
- US-A- 4 506 091

## Beschreibung

Die Erfindung betrifft ein diskontinuierliches Verfahren zur Herstellung von Carbonsäureestern durch Umsetzung von ein-, zwei- oder mehrbasigen Carbonsäuren oder deren Anhydriden mit Alkoholen.

Ester aus mehrbasigen Carbonsäuren, wie beispielsweise Phthalsäure, Adipinsäure, Sebacinsäure und Maleinsäure, und Alkoholen finden weitere Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Bronstedt- oder Lewissäuren, durchgeführt werden. Ganz gleich welche Art der Katalyse gewählt wird, es entsteht immer ein temperaturabhängiges Gleichgewicht zwischen den Einsatzstoffen (Carbonsäure und Alkohol) und den Produkten (Ester und Wasser). Um das Gleichgewicht zu Gunsten des Esters zu verschieben, wird bei vielen Veresterungsverfahren ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann dieses Edukt als Schleppmittel verwendet und nach Abtrennung vom Wasser wieder in den Ansatz zurückgeführt werden. Bei der Veresterung von ein-, zwei- oder mehrbasigen Säuren ist in der Regel der eingesetzte Alkohol das Schleppmittel.

Für viele Anwendungszwecke müssen durch Veresterung einer Carbonsäure hergestellte Ester eine niedrige Säurezahl aufweisen, d. h. die Umsetzung der Carbonsäure sollte praktisch quantitativ erfolgen. Anderenfalls wird die Ausbeute gemindert und die Säure muss, beispielsweise durch Neutralisation, abgetrennt werden. Dies ist aufwendig und kann zu Nebenprodukten führen, die entsorgt werden müssen. Um einen möglichst hohen Umsatz der Carbonsäure zu erhalten, werden Veresterungen in der Regel mit einem Alkoholüberschuss durchgeführt.

Als Veresterungskatalysatoren können Säuren, wie beispielsweise Schwefelsäure oder p-Toluolsulfonsäure, oder Metalle und deren Verbindungen eingesetzt werden. Geeignet sind z. B. Zinn, Titan, Zirkonium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch in der Technik bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirconat.

Nach der erfolgten Veresterung müssen der überschüssige Einsatzalkohol, Säurereste, der Katalysator und/oder seine Folgeprodukte sowie sonstige Nebenprodukte vom gewünschten Carbonsäureester getrennt werden. Die Wirtschaftlichkeit eines Veresterungsverfahren ist nur dann gut, wenn sowohl der Aufwand als auch die Zeit für die Stofftrennung gering sind.

Aus der Literatur sind verschiedene Veresterungsverfahren bzw. Aufarbeitungsverfahren für die so erhaltenen Rohestergemische bekannt.

In DE 19 45 359 wird ein Aufarbeitungsverfahren für Rohestergemische, die durch Umsetzung von Dicarbonsäuren mit 4 bis 12 C-Atomen mit Alkoholen mit 4 bis 16 C-Atomen in Gegenwart metallischer Katalysatoren erhalten wurden, beschrieben, das durch die Abfolge nachstehender Verfahrensschritte gekennzeichnet ist:
a) Die Restsäure im Rohestergemisch wird mit alkalischen Stoffen neutralisiert.
b) Der nicht umgesetzte Alkohol wird durch Wasserdampfdestillation entfernt.
c) Das Produkt wird auf Temperaturen abgekühlt, die unter dem Siedepunkt des Wassers beim jeweiligen Druck liegen.
d) Es werden mindestens 0,5 Massen-% Wasser, bezogen auf das aufzuarbeitende Produkt, zugesetzt.
e) Das Gemisch aus Wasser und aufzuarbeitendem Produkt wird mindestens 15 Minuten bei Temperaturen, die unter der Siedetemperatur des Wassers beim jeweiligen Druck liegen, intensiv gerührt.
f) Das zugesetzte Wasser wird durch Vakuumdestillation entfernt.
g) Der Ester wird filtriert.

In DE 26 12 355 ist ein Aufarbeitungsverfahren für Rohestergemische aus der Veresterung von Phthalsäureanhydrid oder Adipinsäure mit C₆-C₁₃-Alkoholen offengelegt, das folgende Verfahrensschritte umfasst:
a) Das heiße Veresterungsgemisch wird auf Temperaturen unter 100 °C abgekühlt.
b) Das abgekühlte Gemisch wird im Temperaturbereich von 80 bis 100 °C mit wässriger Lauge neutralisiert.
c) Die organische Phase wird durch Dekantieren von der wässrigen Lauge getrennt und mit Wasser gewaschen.
d) Der größte Teil des nicht umgesetzten Alkohols wird abdestilliert.
e) Der Ansatz wird mit Wasserdampf gestrippt.
f) Das Estergemisch wird getrocknet.

Die Neutralisation der abgekühlten Reaktionsmischung erfolgt durch Zugabe von verdünnten Alkalilösungen (0,1 - 5 Gew.-%). Es sind daher recht große Volumina an Base erforderlich (5 - 20 Gew.-%, bezogen auf den Rohester), so dass zur Abtrennung der wässrigen Phase erhebliche Flüssigkeitsmengen bewegt werden müssen. Nach der Neutralisation und Wasserwäsche muss der Ansatz wieder aufgeheizt werden (Zeit- und Energieverlust).

Ein weiteres Aufarbeitungsverfahren für ein Rohestergemisch, erhalten durch Veresterung einer Carbonsäure mit einem Alkohol in Gegenwart einer metallorganischen Verbindung, wird in US 4 304 925 beschrieben. Die Aufarbeitung besteht aus folgenden Verfahrensschritten:
a) Das Rohestergemisch wird auf Temperaturen von unter 100 °C abgekühlt.
b) Zur Hydrolyse der metallorganischen Verbindungen wird der Ansatz mit Wasser versetzt und im Temperaturbereich von 60 bis 98 °C 30 bis 120 Minuten lang gerührt.
c) Zur Neutralisation der Säurereste wird eine Base (Alkalihydroxid, -carbonat oder -hydrogencarbonat) zugesetzt und weitere 30 Minuten lang gerührt.
d) Die Wasserphase wird abgetrennt und die verbleibende organische Phase mit Wasser gewaschen.
e) Durch Wasserdampfdestillation im Vakuum wird der Einsatzalkohol abgetrieben.
f) Nach Zugabe eines Filterhilfsmittel wird der Ansatz filtriert.

US 5 434 294 beschreibt ein Verfahren zur Herstellung von Carbonsäureestern durch Umsetzung von Carbonsäuren mit Alkoholen, bei dem als Katalysator Titan-organische Verbindungen verwendet werden. Die Aufarbeitung des Rohgemisches zum Reinester umfasst folgende Verfahrensschritte:
a) Das Veresterungsgemisch wird auf 80 bis 150 °C abgekühlt.
b) Zur Neutralisation der Säurereste wird eine wässrige Base zugesetzt und 30 Minuten gerührt.
c) Der feuchte neutralisierte Reaktionsansatz wird durch eine Schicht Absorptionsmittel filtriert.
d) Der Einsatzalkohol wird durch Destillation z. B. in einem Dünnschichtverdampfer abgetrennt.

In WO 97/11048 wird eine Aufarbeitungsvorschrift für Veresterungsgemische, hergestellt aus Carbonsäuren und Alkoholen unter Verwendung von organischen Titanverbindungen als Katalysator, offenbart. Die Gewinnung des reinen Esters besteht aus folgenden Stufen:
a) Das Veresterungsgemisch mit einer Säurezahl von 0,56 mg KOH/g wird bei 220 °C durch Zugabe von 10 %iger wässriger Natriumhydrogencarbonat-Lösung neutralisiert und mit einem Schleppmittel wie Toluol versetzt.
b) Es wird auf 90 °C abgekühlt und 2 % Wasser, bezogen auf das Veresterungsgemisch, zugesetzt und gerührt.
c) Wasser, Ausgangsalkohol und sonstige Leichtsieder werden im Vakuum abdestilliert.
d) Der Destillationsrückstand wird filtriert.

In der Produktionsinformation der Hüls AG, "Alkyltitanate als Veresterungskatalysatoren zur Herstellung von Esterweichmachern", 606/93, (September 1993) wird u. a. eine Aufarbeitungsvariante mit folgenden Verfahrensschritten beschrieben:
a) Nach Erreichen einer Säurezahl von 1 mg KOH/g wird der größte Teil des überschüssigen Alkohols durch Vermindern des Drucks bis auf 15 hPa abdestilliert. Dabei nimmt die Säurezahl weiter ab
b) Anschließend werden die Säurereste durch Zugabe von 0,1 % Natriumhydrogencarbonat, bezogen auf das Veresterungsgemisch neutralisiert.
c) Der Ansatz wird bei 150 bis 170 °C und bei einem Vakuum von 10 bis 15 hPa einer Wasserdampfdestillation unterworfen.
d) Nach dem Abkühlen auf 120 bis 100 °C und Zugabe eines Filterhilfsmittel und gegebenenfalls Aktivkohle wird filtriert.

In DE 197 21 347 C2 wird ein Verfahren zur Herstellung von Carbonsäureestern, hergestellt durch Umsetzung von Carbonsäuren mit Alkoholen in Gegenwart organischer Titan- oder Zirkoniumkatalysatoren, beschrieben. Darin ist die Aufarbeitung des rohen Veresterungsgemisches durch folgende Verfahrensschritte gekennzeichnet:
a) Säurereste werden durch Zugabe von einer 5 bis 20%igen Lauge im Überschuss bei der Veresterungsendtemperatur neutralisiert.
b) Der überschüssige Alkohol wird mit Wasserdampf abgetrieben.
c) Der Ansatz wird durch Einleiten eines Inertgases getrocknet.
d) Der Ansatz wird, gegebenenfalls nach Zusatz eines Filterhilfsmittel, filtriert.

In DE 100 43 545 wird ein diskontinuierliches Veresterungsverfahren beschrieben, bei dem das Reaktionswasser durch azeotrope Destillation mit Alkohol aus der Reaktion entfernt wird. Der auf diese Weise ebenfalls entfernte Alkohol wird der Reaktion nach Abtrennung des Wassers im Dekanter wieder zugeführt, so dass das Gleichgewicht der Veresterungsreaktion auf die Produktseite verschoben wird. Auch hier wird nach der Veresterung durch Zugabe von Basen neutralisiert, wobei dies vor oder nach, bevorzugt vor Abdestillation der Hauptmenge des überschüssigen erfolgen kann.

In DE 101 49 350 wird ein Verfahren zur Herstellung von Carbonsäureestern durch Reaktion von Mono-, Di- oder Polycarbonsäuren oder deren Anhydriden mit Alkoholen beschrieben, in dem der überschüssige Alkohol nach der Veresterung entfernt, der so erhaltene Rohester durch Zugabe von 0,005 bis 0,008 mVal einer Base, beispielsweise 25%ige Natronlauge, pro kg Rohester neutralisiert und anschließend filtriert wird, wobei der Alkohol durch mindestens eine Wasserdampfdestillation abgetrennt und die Basenzugabe während der Wasserdampfdestillation erfolgt.

Da die bisherigen Verfahren hinsichtlich der verwendeten Basenmenge, des Dampfverbrauchs und Menge des Filterrückstands nicht voll befriedigten, bestand die Aufgabe, ein verbessertes Verfahren bereitzustellen, welches einen oder mehrere dieser Nachteile nicht aufweist.

Es wurde nun gefunden, dass die Basenmenge bei der Aufarbeitung bei Veresterungsgemischen herabgesetzt werden kann, wenn ein erster Teil der Base, deren molares Verhältnis, gerechnet in Basenäquivalenten, zum Metallatom des Veresterungskatalysators im Bereich von 10 zu 1 bis 1 zu 1 liegt, nach der destillativen Abtrennung des größten Teils des überschüssigen Alkohols, aber vor der Wasserdampfdestillation, zugegeben wird, und dass die zur Neutralisation der Restsäure notwendige Base im Verlauf, z. B. zu Beginn und/oder während der Wasserdampfdestillation bei tieferer Temperatur zugegeben wird.

Gegenstand der Erfmdung ist deshalb ein Verfahren zur Herstellung von Carbonsäureestern durch metallkatalysierte Reaktion von Mono-, Di- oder Polycarbonsäuren oder deren Anhydriden mit Alkohol in Gegenwart eines Überschusses an Alkohol, wobei als Metallkatalysator Titankatalysatoren eingesetzt werden und der überschüssige Alkohol nach der Veresterung entfernt, der so erhaltene Rohester durch Basenzugabe neutralisiert und anschließend filtriert wird, und wobei mindestens ein Teil des überschüssigen Alkohols durch mindestens eine Wasserdampfdestillation entfernt wird, welches dadurch gekennzeichnet, dass
a) in einem ersten Schritt durch Destillation der Alkoholgehalt im Veresterungsgemisch auf einen Anteil von kleiner-gleich 5 Massen-% reduziert wird,
b) dem in Schritt a) erhaltenen Rohester eine erste Menge Base zugesetzt wird, so dass die Base, in Basenäquivalenten gerechnet, mit dem Metallatom des eingesetzten Veresterungskatalysator, im molaren Verhältnis von 10 zu 1 bis 1 zu 1 steht,
c) das in Schritt b) erhaltene Gemisch einer Wasserdampfdestillation unterzogen wird und
d) dass im Verlauf der Wasserdampfdestillation eine zweite Menge Base dem Gemisch zugegeben wird, die mindestens der Menge entspricht, die zur Neutralisation von Restsäure notwendig ist.

Erhalten wird eine Zusammensetzung, enthaltend einen Vollester einer Mono-, Di- oder Polycarbonsäure, einen Alkohol, der dem mit der Mono-, Di- oder Polycarbonsäure oder dessen Anhydrid zum Ester umgesetzten Alkohol entspricht, und Wasser, welche dadurch gekennzeichnet ist, dass der Anteil des Vollesters größer oder gleich 99,5 Massen-%, der Anteil des Alkohols kleiner oder gleich 100 Massen-ppm und der Anteil an Wasser kleiner oder gleich 0,04 Massen-% beträgt.

Das erfindungsgemäße Verfahren weist gegenüber bekannten Verfahren folgende Vorteile auf: Die bei der Neutralisation der Restsäure als Nebenreaktion ablaufende Verseifung des Esters kann stark reduziert werden. Durch die geringere Verseifung liegt im Reaktionsgemisch weniger Säure vor bzw. entsteht weniger Säure, weshalb die zur Neutralisation benötigte Menge an Base geringer ist als bei bekannten Verfahren.

Dadurch, dass weniger Säure im Veresterungsgemisch vorhanden ist bzw. entsteht, die neutralisiert werden muss, entstehen auch weniger Neutralisationsprodukte, die als festes Salz abgetrennt werden müssen. Dies hat den Vorteil, dass die Abtrennzeiten (Filtrierzeiten) geringer sind und wegen des geringeren Filterrückstands geringere Entsorgungskosten anfallen können.

Durch die Vermeidung der Verseifung wird als weiterer Vorteil erreicht, dass sich der Säureeinsatzfaktor für die Estersynthese verbessert. Durch die Verringerung der Rückbildung von Alkohol durch Verseifung wird außerdem die zur Abtrennung von Restalkoholspuren benötigte Energie geringer.

Nachfolgend wird die Erfindung beispielhaft beschrieben.

Das erfindungsgemäße Verfahren zur Herstellung von Carbonsäureestern durch metallkatalysierte Reaktion von Mono-, Di- oder Polycarbonsäuren oder deren Anhydriden mit Alkohol in Gegenwart eines Überschusses an Alkohol, wobei der überschüssige Alkohol nach der Veresterung entfernt, der so erhaltene Rohester durch Basenzugabe neutralisiert und anschließend filtriert wird, und wobei zumindest ein Teil des überschüssigen Alkohols durch mindestens eine Wasserdampfdestillation entfernt wird, zeichnet sich dadurch aus, dass
a) in einem ersten Schritt, vorzugsweise nach Beendigung der Veresterungsreaktion, durch Destillation der Alkoholgehalt im Veresterungsgemisch auf einen Anteil von kleiner-gleich 5 Massen-% reduziert wird,
b) dem in Schritt a) erhaltenen Rohester eine erste Menge Base zugesetzt wird, so dass die Base, in Basenäquivalenten gerechnet, mit dem Metallatom des eingesetzten Veresterungskatalysator im molaren Verhältnis von 10 zu 1 bis 1 zu 1 steht,
c) das in Schritt b) erhaltene Gemisch einer Wasserdampfdestillation unterzogen wird und
d) dass zu Beginn und/oder im Verlauf der Wasserdampfdestillation eine zweite Menge Base dem Gemisch zugegeben wird, die mindestens der Menge entspricht, die zur Neutralisation von Restsäure notwendig ist.

Das erfindungsgemäße Verfahren zur Herstellung von Carbonsäureestern erfolgt in der Weise, dass durch eine metallkatalysierte Reaktion Mono-, Di- oder Polycarbonsäuren oder deren Anhydride mit Alkohol in Gegenwart eines Überschusses an Alkohol umgesetzt werden. Diese Umsetzung kann dabei wie im Stand der Technik, insbesondere wie in DE 101 49 350 und DE 100 43 545 beschrieben erfolgen. Bezüglich der Umsetzung wird ausdrücklich auf die Beschreibung von DE 101 49 350 und DE 100 43 545 verwiesen.

Bevorzugt werden bei der Umsetzung als Säurekomponente Mono-, Di- und Polycarbonsäuren sowie deren Anhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden. Die Säuren können aliphatisch, carbocyclisch, heterocyclisch, gesättigt oder ungesättigt sowie aromatisch sein. Aliphatische Carbonsäuren besitzen mindestens 4 C-Atome. Beispiele für aliphatische Carbonsäuren bzw. deren Anhydride sind: Bernsteinsäure, Bernsteinsäureanhydrid, Korksäure, Azelainsäure, Decandisäure, Dodecandisäure, Brassylsäure. Beispiele für carbocyclische Verbindungen sind: Hexahydrophthalsäureanhydrid, Hexahydrophthalsäure, Cyclohexan-1,4-dicarbonsäure, Cyclohex-4-en-1,2-dicarbonsäure, Cyclohexen-1,2-dicarbonsäurcanhydrid, 4-Methylcyclohexan-1,2-dicarbonsäure, 4-Methylcyclohexan-1,2-dicarbonsäurcanhydrid, 4-Methylcyclohex-4-en-1,2-dicarbonsäure, 4-Methylcyclohex-4-en-1,2-dicarbonsäureanhydrid. Beispiele für aromatische Verbindungen sind: Benzoesäure, Phthalsäure, Phthalsäureanhydrid, Isophthalsäure, Therephthalsäure, Trimellitsäure, Trimesinsäure, Pyromellitsäure, Pyromellitsäureanhydrid oder Naphthalindicarbonsäuren. Bevorzugt eingesetzte Säuren sind Phthalsäure, 1,2-Cyclohexandicarbonsäure und/oder Trimellitsäure und/oder deren Anhydride. Werden Säuren verwendet, die cis-trans-Isomere aufweisen, können diese Säuren isomerenrein oder als Gemische von cis- und trans-Isomeren eingesetzt werden.

Als alkoholische Komponente werden bevorzugt verzweigte oder lineare aliphatische Alkohole mit 4 bis 13 C-Atomen eingesetzt. Die Alkohole sind bevorzugt einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden können, sind beispielsweise n-Butanol, Isobutanol, n-Octanol (1), n-Octanol(2), 2-Ethylhexanol, Nonanole, Decylalkohole oder Tridecanole. Solche Alkohole können insbesondere durch Hydroformylierung von Olefinen oder Aldolkondensation von Aldehyden und jeweils anschließende Hydrierung hergestellt werden. Die Alkohole können als reine Verbindung, als Gemisch isomerer Verbindungen oder als Gemisch von Verbindungen mit unterschiedlicher C-Zahl eingesetzt werden.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägern eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Cornils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Process., Int. Ed. (1986) 65 (2. Sect. 1), Seite 31-33, EP 0 395 857).

Ganz besonders bevorzugte Einsatzstoffe für die erfindungsgemäße Veresterung sind Gemische isomerer Nonanole (Isononanol) oder Gemische isomerer Tridecanole (Isotridecanol). Diese können bevorzugt durch Oligomerisierung linearer Butene zu C₈-Olefinen und C₁₂-Olefinen nach dem Octol-Prozess, mit anschließender Hydroformylierung und Hydrierung hergestellt werden.

Als Veresterungskatalysatoren werdenTitanverbindungen, insbesondere Tetra(isopropyl)orthotitanat oder Tetra(butyl)orthotitanat eingesetzt.

Bei der erfindungsgemäßen Herstellung von Carbonsäureestern beträgt die Katalysatorkonzentration zu Beginn der Veresterungsreaktion vorzugsweise von 0,005 bis 1,0 Massen-%, besonders bevorzugt von 0,0075 bis 0,1 Massen-% bezogen auf das Gemisch aller Einsatzstoffe (Reaktionsgemisch vor destillativer Abtrennung einer Komponente).

Der umzusetzende Alkohol, der auch als Schleppmittel dient, wird im stöchiometrischen Überschuss, bevorzugt 5 bis 50 %, besonders bevorzugt 10 bis 30 % der stöchiometrisch notwendigen Menge eingesetzt.

Die Reaktionstemperaturen betragen bei der metallkatalysierten Veresterung vorzugsweise von 160 °C bis 270 °C, bevorzugt von 180 bis 250 °C. Die optimalen Temperaturen hängen von den Einsatzstoffen, dem Reaktionsfortschritt und der Art des Katalysators ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Normaldruck oder leichtem Überdruck und bei höher siedenden Alkoholen bei verminderten Druck durchgeführt. Beispielsweise wird bei der Umsetzung von Phthalsäureanhydrid mit einem Gemisch isomerer Nonanole vorzugsweise in einem Temperaturbereich von 170 °C bis 250 °C im Druckbereich von 0,1 MPa bis 10 hPa gearbeitet.

Es ist möglich, das während der Veresterungsreaktion entstandene Reaktionswasser durch azeotrope Destillation während der laufenden Veresterungsreaktion zu entfernen. Durch diese optionale Maßnahmen wird nicht nur das Reaktionswasser, sondern auch ein Teil des Alkohols entfernt. Vorzugsweise wird die durch die azeotrope Destillation aus der Reaktion entfernte Flüssigkeitsmenge wieder ergänzt.

Die in die Reaktion zurückzuführende Flüssigkeitsmenge kann teilweise oder vollständig aus dem Einsatzalkohol bestehen. Dieser Alkohol kann durch Aufarbeitung des azeotropen Destillats gewonnen werden. Es ist auch möglich, die Aufarbeitung zu einem späteren Zeitpunkt durchzufähren und die entfernte Flüssigkeitsmenge ganz oder teilweise durch frischen Alkohol, d. h. aus einem in einem Vorratsgefäß bereitstehenden Alkohol zu ersetzen.

Durch die azeotrope Destillation wird während der Reaktion ein Alkohol-Wasser-Gemisch als Azeotrop aus der Reaktionsmischung abdestilliert. Die Brüden können z. B. über eine kurze Kolonne (Einbauten oder Füllkörper; 1 bis 5, vorzugsweise 1 bis 3 theoretische Böden) das Reaktionsgefäß verlassen und werden anschließend kondensiert. Das Kondensat kann in eine wässrige und in eine alkoholische Phase getrennt werden. Zur Trennung und/oder zur Kondensation kann eine Kühlung erforderlich sein. Die wässrige Phase wird abgetrennt und kann, gegebenenfalls nach Aufarbeitung, verworfen oder als Strippwasser bei einer Nachbehandlung des Esters verwendet werden.

Die alkoholische Phase, die nach Trennung des azeotropen Destillat anfällt, kann teilweise oder vollständig in das Reaktionsgefäß zurückgeführt werden. In der Praxis hat sich eine standgeregelte Füllstandskontrolle der Reaktion zur Zufuhr des Alkohols bewährt. Optional kann frischer Alkohol zur abgetrennten Alkoholphase, die in die Reaktion zurückgeführt wird, beigefügt werden.

Für die Zufuhr des Alkohols in die Veresterungsreaktion gibt es verschiedene Möglichkeiten. Der Alkohol kann beispielsweise als Rückfluss auf die Kolonne gegeben werden. Eine andere Möglichkeit ist, den Alkohol, gegebenenfalls nach Aufheizung, in das flüssige Reaktionsgemisch zu pumpen. Durch die Abtrennung des Reaktionswasser sinkt das Reaktionsvolumen in der Apparatur. Es kann jedoch vorteilhaft sein, wie in DE 100 43 545 beschrieben (kürzere Reaktionszeit) während der Reaktion so viel Alkohol nachzuspeisen, wie dem Volumen des abgetrennten Destillats (Wasser und gegebenenfalls Alkohol) entspricht, sodass der Füllstand im Reaktionsgefäß konstant bleibt. Durch die Vergrößerung des Alkoholüberschusses wird das Gleichgewicht zu Gunsten der Vollester verschoben.

Die Veresterung wird vorzugsweise in einem Reaktionsgefäß durchgeführt, in dem der Reaktionsansatz mit Hilfe eines Rührers oder einer Umlaufpumpe intensiv vermischt werden kann. Die Edukte und der Katalysator können gleichzeitig oder hintereinander in den Reaktor eingefüllt werden. Ist einer der Einsatzstoffe bei der Einfülltemperatur fest, ist es zweckmäßig, die flüssige Einsatzkomponente vorzulegen. Feste Einsatzstoffe können als Pulver, Granulat, Kristallisat oder Schmelze eingespeist werden. Um die Chargenzeit zu verkürzen, kann es vorteilhaft sein, während des Einfüllens mit dem Aufheizen zu beginnen. Der Katalysator kann in reiner Form oder als Lösung, bevorzugt gelöst in einem der Einsatzstoffe, zu Beginn oder erst nach Erreichen der Reaktionstemperatur eingebracht werden.

Nach Beendigung der Reaktion kann das Reaktionsgemisch, das im Wesentlichen aus Vollester (Zielprodukt) und überschüssigem Alkohol besteht, neben dem Katalysator und/oder dessen Folgeprodukte auch noch geringe Mengen an Estercarbonsäure(n) (Halbester) und/oder nicht umgesetzter Carbonsäure und gegebenenfalls Zersetzungsprodukte des Alkohols wie beispielsweise Olefine enthalten.

Aus dem so erhaltenen Veresterungsgemisch wird in einem ersten Schritt a) durch Destillation soviel des überschüssigen Alkohols entfernt, dass der Alkoholgehalt im Veresterungsgemisch auf einen Anteil von kleiner-gleich 5 Massen-%, vorzugsweise kleiner-gleich 2,5 Massen-%, bevorzugt von 2,5 bis 0,1 Massen-% und besonders bevorzugt von 1 bis 0,5 Massen-% reduziert wird. Es kann vorteilhaft sein, die in Schritt a) durchgeführte Destillation nur bis zu einem minimalen Alkoholgehalt von 0,1 Massen-%, vorzugsweise 0,5 Massen-% durchzufähren, da bei dem Versuch den Alkoholgehalt bereits in Schritt a) auf deutlich kleinere Werte zu reduzieren häufig eine teilweise Zersetzung des gebildeten Esters zu beobachten ist. Diese Destillation gemäß Schritt a) ist vorzugsweise keine Wasserdampfdestillation. Die Destillation kann ohne oder mit dem Vorsehen eines Rücklaufs durchgeführt werden. Ein Rücklauf ist insbesondere dann vorteilhaft, wenn die Siedetemperaturen des verwendeten Alkohols und des Esters nur geringfügig auseinanderliegen. So kann bei der Destillation von Isononanol aus einem Veresterungsgemisch, welches Düsononylphthalat als Zielprodukt enthält, ohne Rücklauf abgetrennt werden. Bei der Abtrennung von Isononanol aus einem Veresterungsgemisch, welches Isononylbenzoat als Zielprodukt aufweist, kann es hingegen vorteilhaft sein, einen Rücklauf vorzusehen, damit die Abtrennung möglichst vollständig gelingt. Bevorzugt kann die Destillation gemäß Schritt a) so durchgeführt werden, dass zur Destillation eine mit dem Reaktionsgefäß in Verbindung stehende kurze Kolonne, die Einbauten oder Füllkörper und 1 bis 5, vorzugsweise 2 bis 3 theoretische Böden aufweist, eingesetzt wird. Dies ist insbesondere dann vorteilhaft, wenn das Veresterungsgemisch Isononylbenzoat als Zielprodukt enthält. Der Alkoholgehalt kann während der Destillation mittels einer gaschromatischen Analyse (GC) bestimmt werden. Die Temperatur und der Druck, bei denen die Destillation durchgeführt werden sollte, ist abhängig von den bei der Veresterung eingesetzten Komponenten und kann auf einfache Weise durch Vorversuche bestimmt werden.

Im erfindungsgemäßen Verfahren wird in Schritt b) zunächst der zur Veresterung eingesetzte Metallkatalysator bzw. das daraus entstandene Derivat durch Zusatz einer ersten Menge an Base desaktiviert. Auch diese Menge kann in Bezug auf das Metall im Metallkatalysator bis zu einem molaren Überschuss von 10 zugegeben werden. Die erste Menge an Base ist aber so gewählt, dass eine vollständige Neutralisation des Veresterungsgemisches nicht erfolgt. Die Zugabe der ersten Menge an Base erfolgt vor der Wasserdampfdestillation zur Abtrennung des überschüssigen Alkohols. Die Zugabe einer oder mehrerer weiterer Mengen an Basen (Schritt d)) erfolgt erst im Verlauf der Wasserdampfdestillation.

Als Basen können in dem erfindungsgemäßen Verfahren Verbindungen der Alkalimetalle und/oder Erdalkalimetalle verwendet werden, wie beispielweise deren Hydroxide, Carbonate oder Hydrogencarbonate. Es ist auch möglich Gemische verschiedener Basen zu verwenden. Die Base kann in fester Form oder bevorzugt als Lösung, insbesondere als wässrige Lösung, eingesetzt werden. Wenn eine wässrige Lösung einer Base eingesetzt wird, beträgt der Gehalt der basischen Verbindung in der wässrigen Lösung von 1 bis 30 Massen-%, besonders bevorzugt von 5 bis 30 Massen-% und besonders bevorzugt von 20 bis 25 Massen-%. Besonders bevorzugt wird im erfindungsgemäßen Verfahren Natronlauge (wässrige Lösung von Natriumhydroxid), bevorzugt eine 22 bis 25 Massen-%ige Natronlauge als Base eingesetzt.

Im erfindungsgemäßen Verfahren wird in Schritt b) dem Rohester eine erste Menge Base zugesetzt wird, so dass die Base, in Basenäquivalenten gerechnet, mit dem Metallatom des eingesetzten Veresterungskatalysator im molaren Verhältnis von 10 zu 1 bis 1 zu 1 steht. Bevorzugt wird bei der ersten Basenzugabe in Schritt b) soviel Base zugegeben, dass die Base in Basenäquivalenten gerechnet mit dem Metallatom des Veresterungskatalysators im molaren Verhältnis von 8 zu 1 bis 2 zu 1 und besonders bevorzugt von 6 zu 1 bis 4 zu 1 steht.

Bei Einsatz einer einwertigen Base, wie beispielsweise Natriumhydroxid, liegt dementsprechend das molare Verhältnis zwischen Base und Metallatom des Veresterungskatalysators im Bereich von 10 zu 1 bis 1 zu 1, bevorzugt im Bereich von 8 zu 1 bis 2 zu 1 und besonders bevorzugt im Bereich von 6 zu 1 bis 4 zu 1. Bei Einsatz einer zweiwertigen Base liegt dementsprechend das molare Verhältnis zwischen Base und Metallatom des Veresterungskatalysators im Bereich von 5 zu 1 bis 0,5 zu 1, bevorzugt im Bereich von 4 zu 1 bis 1 zu 1 und besonders bevorzugt im Bereich von 3 zu 1 bis 2 zu 1.

Bei der Verwendung eines Tetraalkylmetallkatalysators, wie z. B. Tetra(butyl)orthotitanat, als Veresterungskatalysator beträgt das molare Verhältnis zwischen Base und Metallatom des Veresterungskatalysators bei Verwendung einer einwertigen Base bevorzugt zumindest 4 zu 1 und bei einer zweiwertigen Base zumindest 2 zu 1.

Bevorzugt, insbesondere bei der Diisononylphthalat-Herstellung, erfolgt die Zugabe der ersten Menge an Base in Schritt b) bei Temperaturen des Rohesters von 230 bis 200 °C, insbesondere von 220 bis 205°C. Die Zugabedauer beträgt vorzugsweise von 1 bis 30 Minuten, insbesondere 5 bis 15 Minuten. Während der Zugabe wird der Rohester innig durchmischt, z. B. durch Umwälzen oder Rühren des Inhalts des Gefäßes in dem die Zugabe der ersten Menge der Base erfolgt.

Nach Beendigung der Zugabe der ersten Menge an Base, vorzugsweise nach einer Reaktionszeit von 0 bis 10 Minuten, bevorzugt von 1 bis 5 Minuten, nach Beendigung der Zugabe der ersten Menge an Base wird das Reaktionsgemisch gemäß Schritt c) einer Wasserdampfdestillation unterworfen. Es kann vorteilhaft sein, wenn die Wasserdampfdestillation im Temperaturbereich 220 bis 110 °C, insbesondere im Bereich von 210 °C bis 130 °C erfolgt (Temperatur im Reaktor). Während der Wasserdampfdestillation kann es vorteilhaft sein, die Temperatur zu senken, vorzugsweise in der Weise, dass die Wasserdampfdestillation am oberen Bereich oder Wert des angegebenen Temperaturbereichs gestartet wird und zum Ende der Wasserdampfdestillation eine Temperatur erreicht wird, die dem unteren Bereich oder Wert des angegebenen Temperaturbereichs entspricht. Vorzugsweise wird die Temperatur während der Wasserdampfdestillation mit einer Geschwindigkeit von 3 bis 60 °C/h, bevorzugt mit einer Geschwindigkeit von 6 bis 30 °C/h und besonders bevorzugt mit einer Geschwindigkeit von 10 bis 25 °C/h gesenkt.

Die Temperatur kann über den Energiegehalt des eingebrachten Wassers oder Dampfes sowie durch Wärmeaustausch (z. B. mittels Wärmetauscher) reguliert werden. Der Destillationsdruck während der Wasserdampfdestillation beträgt vorzugsweise von 50 bis 500 hPa_{absolut}, insbesondere von 80 bis 150 hPa_{absolut}.

Bei der Wasserdampfdestillation werden vorzugsweise je Tonne Rohester 5 bis 100 kg/h Wasser (flüssig und/oder dampfförmig), insbesondere 10 bis 50 kg/h eingesetzt. Der spezifische Wassereinsatz (kg/(t*h) kann während der Wasserdampfdestillation konstant oder variabel sein. Vorzugsweise bleibt der spezifische Wassereinsatz während der Wasserdampfdestillation konstant.

Nach Ende der Zugabe der ersten Menge an Base wird die Säurezahl der Reaktionsmischung gemäß DIN EN ISO 2114 bestimmt und aus der Differenz der gemessenen Säurezahl und einer spezifizierten Säurezahl, vorzugsweise < 0,05 mg KOH/g, bevorzugt < 0,04 mg KOH/g, sowie der Masse des Reaktionsgemisches die Basenmenge berechnet, die im Verlauf der Wasserdampfdestillation zum Zwecke der Neutralisation zugegeben werden muss. Vorzugsweise erfolgt die Bestimmung der Säurezahl 5 bis 60 Minuten, bevorzugt 10 bis 30 Minuten nach dem Ende der Zugabe der ersten Menge an Base.

Im Verlauf der Wasserdampfdestillation wird gemäß Schritt d) dem in der Wasserdampfdestillation vorliegenden Gemisch eine zweite Menge Base zugegeben, die mindestens der Menge entspricht, die zur Neutralisation von Restsäure notwendig ist.

Vorzugsweise wird die, nach Basenäquivalenten gerechnet, dem Ein- bis Dreifachen der berechneten Basenmenge, bevorzugt dem Eineinhalb- bis Zweifachen der berechneten Basenmenge entspricht, dem Gemisch zugegeben. Bevorzugt wird die gleiche Base, die bei ersten Zugabe verwendet wurde, eingesetzt, es ist jedoch auch möglich, eine andere Base zu verwenden.

Die Zugabe der zweiten Menge Base in Schritt d) kann in ein, zwei oder mehr Schritten erfolgen. Vorzugsweise erfolgt die Zugabe in einem Schritt. Es kann vorteilhaft sein, wenn zumindest eine Teilmenge der in Schritt d) zugegebenen zweiten Menge an Base zu Beginn der Wasserdampfdestillation zugegeben wird. Bevorzugt wird die gesamte Menge der zweiten Menge an Base zu Beginn der Wasserdampfdestillation dem Gemisch zugegeben.

Es kann vorteilhaft sein, nach der Zugabe der zweiten Menge an Base, vorzugsweise 1 bis 30 Minuten, bevorzugt 5 bis 15 Minuten nach dem Ende der Zugabe der zweiten Menge an Base die Säurezahl des Reaktionsgemisches erneut zu bestimmen. Liegt die Säurezahl weiterhin oberhalb der Spezifikation, wird bevorzugt ein weiteres Mal durch Zugabe von Base zum Reaktionsgemisch neutralisiert. Vorzugsweise wird eine Menge Base zugegeben, die mindestens der Menge entspricht, die zur Neutralisation von Restsäure notwendig ist. Vorzugsweise wird die, nach Basenäquivalenten gerechnet, dem Ein- bis Dreifachen der berechneten Basenmenge, bevorzugt dem Eineinhalb- bis Zweifachen der berechneten Basenmenge entspricht, dem Gemisch zugegeben. Bevorzugt wird die gleiche Base, die bei ersten Zugabe verwendet wurde, eingesetzt, es ist jedoch auch möglich, eine von der ersten Base verschiedene Base zu verwenden. Dieser Vorgang aus Bestimmung der Säurezahl und erneuter Zugabe von Base kann so oft wiederholt werden, bis das Reaktionsgemisch die gewünschte Spezifikation aufweist.

Die bei der erfindungsgemäßen Veresterung erhaltenen Veresterungsgemische werden, wie bereits beschrieben, nach dem erfindungsgemäßen Verfahren aufgearbeitet. Der aus der Wasserdampfdestillation erhaltene Ester weist vorzugsweise einen Wassergehalt von deutlich kleiner 0,1 Massen-%, bevorzugt kleiner 0,05 Massen-%, besonders bevorzugt von kleiner-gleich 0,025 Massen-% und besonders bevorzugt von 0,005 bis 0,025 Massen-%. Optional kann das aus der Wasserdampfdestillation als Sumpfprodukt erhaltene Gemisch einer weiteren Trocknung zugeführt werden. Dies kann insbesondere dann vorteilhaft sein, wenn das aus der Wasserdampfdestillation als Sumpfprodukt erhaltene Gemisch noch einen Wassergehalt im Bereich von 0,1 Massen-% bis 0,05 Massen-% aufweist. Die Trocknung kann z. B. in der Weise erfolgen, dass das Gemisch bei einer Temperatur von 180 °C bis 130 °C, insbesondere 150 bis 130 °C bei einem Druck von 80 bis 30 hPa, insbesondere 60 bis 40 hPa, mit oder ohne Verwendung von Inertgas entwässert wird. Bei der Verwendung von Inertgas kann dieses z. B. über Düsen durch das Reaktionsgemisch geschickt werden. Die Trocknung kann beendet werden, wenn der Wassergehalt auf den gewünschten Wert von kleiner-gleich 0,05 Massen-% gesenkt wurde. Für die Trocknung von Diisononylphthalat mit einem Wassergehalt von ca. 0,1 Massen-% auf einen Wassergehalt von ca. 0,05 Massen-% werden beispielsweise, wenn die Trocknung durch Rühren bei 130 °C unter einem Vakuum von 50 hPa_{absolut} erfolgt, in der Regel weniger als 10 Minuten Trockenzeit benötigt.

Es kann vorteilhaft sein, aus dem als Sumpfprodukt der Wasserdampfdestillation, mit oder ohne vorangegangener Trocknung, Feststoffe, wie z. B. die durch Neutralisation der Restsäuren entstandenen Salze oder die durch Zerstörung des Metallkatalysators entstandenen Feststoffe, zu entfernen. Das Entfernen kann vor oder nach der Trocknung erfolgen. Vorzugsweise erfolgt das Entfernen der Feststoffe nach der Trocknung, da die Entfernung der Feststoffe, wenn noch eine Restfeuchte vorhanden ist, häufig schwieriger ist, weil sich der verwendete Filter leichter zusetzt. Das Entfernen der Feststoffe kann, gegebenenfalls nach Zusatz eines Filterhilfsmittel, durch filtrieren vorgenommen werden. Optional können die Feststoffe durch Zentrifugieren abgetrennt werden. Die Stofftrennung kann bei einer Temperatur von 20 bis 150 °C erfolgen.

Die Ester aus ein-, zwei- oder mehrbasigen Carbonsäuren und Alkoholen, hergestellt gemäß dem erfindungsgemäßen Verfahren, können Anwendung in Lackharzen, als Bestandteile von Anstrichmitteln und insbesondere als Weichmacher für Kunststoffe finden. Bevorzugte Weichmacher für PVC sind insbesondere Düsononyl- oder Dioctylphthalate und Diisononyl- oder Dioctylhexahydrophthalate. Weiterhin können die erfindungsgemäß hergestellten Ester als Komponente von Schmierstoffen Verwendung finden.

Eine Zusammensetzung, enthaltend einen Vollester einer Mono-, Di- oder Polycarbonsäure, einen Alkohol, der dem mit der Mono-, Di- oder Polycarbonsäure oder deren Anhydrid zum Ester umgesetzten Alkohol entspricht, und Wasser, welche dadurch gekennzeichnet ist, dass der Anteil des Vollesters größer oder gleich 99,5 Massen-%, der Anteil des Alkohols kleiner oder gleich 200 Massen-ppm und der Anteil an Wasser kleiner oder gleich 0,04 Massen-% beträgt, ist z. B. nach dem erfindungsgemäßen Verfahren erhältlich.

Es kann vorteilhaft sein, wenn der Anteil des Vollesters größer oder gleich 99,95 Massen-%, der Anteil des Alkohols kleiner oder gleich 100 Massen-ppm und der Anteil an Wasser kleiner oder gleich 0,025 Massen-% beträgt. Besonders bevorzugt beträgt der Anteil des Vollesters von 99,95 bis 99,97 Massen-%, der Anteil des Alkohols von 10 bis 100 Massen-ppm und der Anteil an Wasser 0,01 bis 0,025 Massen-%. Die Zusammensetzungen haben den Vorteil, dass sie direkt, ohne weitere Aufarbeitung in den Anwendungen der Vollester eingesetzt werden können. Auf Grund der Zusammensetzung der Zusammensetzungen wird eine Hydrolyse der Ester weitestgehend vermieden. Bei der Verarbeitung von Kunststoffzusammensetzungen, wie z. B. PVC-Zusammensetzungen, die die Zusammensetzungen aufweisen, kann eine Bläschenbildung durch Verdampfung von in der Zusammensetzung enthaltendem Wasser oder Alkohol weitestgehend vermieden werden.

Bevorzugt weist die Zusammensetzung als Vollester ein Alkylbenzoat, Dialkylphthalat, Dialkyladipat, Dialkylhexahydrophthalat, Dialkylterephthalat oder Dialkylisophthalat und als Alkohol, einen Alkohol mit einer entsprechenden Alkylgruppe auf. Ganz besonders bevorzugt sind Zusammensetzungen, bei denen der Vollester ein Isononylbenzoat, Diisononylphthalat oder ein Diisononylhexahydrophthalat und der Alkohol Isononanol ist. Ebenso bevorzugt sind Zusammensetzungen, bei denen der Vollester ein Diisotridecylphthalat oder ein Diisotridecylhexahydrophthalat und der Alkohol Isotridecanol ist.

Die Zusammensetzung kann gegebenenfalls noch eine Metallverbindung, ausgewählt aus den Verbindungen von Titan, Zirkonium und Zinn in einem Anteil von 0,005 bis 1 Massen-% aufweisen. Ist die Zusammensetzung zur Abtrennung der Metallverbindung behandelt worden, z. B. einer Filtration oder Destillation unterzogen worden, kann die Zusammensetzung aber auch keine Metallverbindung (also unterhalb der Nachweisgrenze) oder von 10 Massen-ppm bis 0,005 Massen-% an Metallverbindung, ausgewählt aus den Verbindungen von Titan, Zirkonium und Zinn enthalten.

Die Zusammensetzung kann des weiteren Salze der eingesetzten Mono-, Di- oder Polycarbonsäure, insbesondere Natriumsalze aufweisen. Die Zusammensetzung kann aber auch frei oder nahezu frei an solchen Salzen sein. Bevorzugt ist die Zusammensetzung frei oder nahezu frei an solchen Salzen, wobei die Salze vorzugsweise im Rahmen der Abtrennung der Metallverbindungen von Zirkonium, Titan oder Zinn abgetrennt werden.

Die vorgenannten Zusammensetzungen, insbesondere die Zusammensetzungen als Vollester Düsononylphthalat oder Düsononylhexahydrophthalat und als Alkohol Isononanol aufweisen oder die als Vollester Düsotridecylphthalat oder Diisotridecylhexahydrophthalat und als Alkohol Isotridecanol aufweisen, können in Lackharzen, als Bestandteile von Anstrichmitteln und insbesondere als Weichmacher für Kunststoffe verwendet werden. Bevorzugt werden die Zusammensetzungen als Weichmacher für PVC verwendet. Weiterhin können die Zusammensetzungen als Komponente von Schmierstoffen Verwendung finden.

Nachfolgend wird das erfindungsgemäße Verfahren beispielhaft für eine Veresterung von Phthalsäureanhydrid beschrieben. Wird z. B. Phthalsäureanhydrid mit Isononanol oder 2-Ethylhexanol umgesetzt, so erfolgt die Veresterung bevorzugt bei einer Temperatur von 180 bis 250 °C, optional unter ständiger Wasserentfernung durch Azeotropdestillation. In einer Ausfiümmgsform erfolgt dies so lange, bis die Säurezahl auf kleiner 0,5 gesunken ist. Dann werden unter Verringerung des Druckes, beispielsweise bis auf 15 hPa, der überschüssige Alkohol, geringe Mengen an Reaktionswasser und gegebenenfalls andere Leichtsieder abdestilliert. Durch die dabei fast vollständige Entfernung des Reaktionswassers steigt der Säureumsatz und die Säurezahl sinkt noch deutlich weiter ab. Während des Abdestillieren kann auf Zufuhr von Energie verzichtet werden, um die Temperatur des Veresterungsgemisches vor der Neutralisation zu senken. Werden andere Säuren bzw. Alkohole eingesetzt, müssen die Reaktionstemperaturen, -drücke und -zeiten entsprechend angepasst werden.

Die folgenden Beispiele sollen die Erfindung näher erläutern.

### Beispiel 1: Herstellung eines Rohestergemisches

Der verwendete Veresterungsreaktor besteht aus einem Rührbehälter mit Heizschlange (4 MPa Dampf), einem Trennsystem aus Destillationskolonne und Trennbehälter für die Reaktionswasser/Alkohol-Trennung und einer Rücklaufleitung für den Überschussalkohol. Die Apparatur wird vor Befüllung mit Stickstoff sauerstofffrei gespült.

Einsatzmengen:
1000 kg Phthalsäureanhydrid (flüssig)
2430 kg Isononanol (OXENO Olefinchemie GmbH)
0,7 kg Butyltitanat (Rhone-Poulenc)

Nachdem zunächst 400 kg Isononanol im Reaktor vorgelegt worden waren, wurde mit dem Aufheizen unter ständigem Rühren begonnen. Während des Aufheizens wurden Phthalsäureanhydrid in flüssiger Form und die Restalkoholmenge (2030 kg) gleichzeitig getrennt eingespeist. Nachdem das Reaktionsgemisch eine Temperatur von 160 °C erreicht hatte, wurde der Titankatalysator zugegeben. Bei 180 °C begann das Gemisch zu sieden. Während der Veresterung wurde Wasser freigesetzt und als Isononanol-Wasser-Azeotrop abdestilliert. Die Säurezahl der Reaktionsmischung sank von einem Startwert von 100 mg KOH/g bei Siedebeginn auf 10 mg KOH/g nach 150 Minuten, 1 mg KOH nach 290 Minuten und 0,5 mg KOH/g nach 330 Minuten (hier und in den nachfolgend aufgeführten Beispielen bestimmt gemäß DIN EN ISO 2114, nach dem kolorimetrischen Titrationsverfahren gemäß Verfahren A, wobei als Lösemittel ein Toluol/Isopropanol/Wasser-Gemisch mit einem Volumenverhältnis von 1 zu 1,5 zu 0,2 eingesetzt wurde). Der Anlagendruck war zu diesem Zeitpunkt stufenweise bis auf 600 hPa verringert worden und die Reaktionstemperatur betrug 240 °C, um den gewünschten Alkoholrücklauf, der so groß war, dass die abdestillierte Azeotrop-Menge durch den Alkoholrücklauf annähernd (gegebenenfalls durch unter Zugabe von Frischalkohol) ersetzt werden kann, zur effektiven Wasserabtrennung aufrechterhalten zu können. Der Alkoholgehalt im Reaktionsgemisch lag am Ende der Veresterung bei 6,5 Massen-%.

### Beispiel 2 (Vergleichsbeispiel für die Aufarbeitung)

In dem Reaktor, in dem sich das nach Beispiel 1 hergestellte Rohestergemisch befand, wurde nun der Anlagedruck von 600 hPa auf 10 hPa gesenkt und der Alkoholgehalt durch Destillation auf 1 Massen-% verringert. Dabei wurde die Verdampfungsenergie dem Reaktor durch Dampfbeheizung zugeführt um die Temperatur bei 240 °C zu halten. Anschließend wurde bei gleichmäßig sinkender Temperatur im Temperaturbereich 240 bis 220 °C eine Stunde lang einer Wasserdampfdestillation (12 kg Wasser pro h und to Rohester) unterworfen. Bei 220 °C wurde eine Menge von 5,334 kg einer 25 Massen-%igen wässrigen Natronlauge zugegeben. Anschließend wurde bei gleichmäßig sinkender Temperatur die Wasserdampfdestillation bis zu einer Temperatur von 140 °C fortgesetzt (Dauer 2 h). Während der Wasserdampfdestillation wurde bei 190 °C die Säurezahl gemäß DIN EN ISO 2114 wie in Beispiel 1 beschrieben bestimmt und der Ansatz bei 180 °C mit 0,7 kg einer 25 Massen-%igen wässrigen Natronlauge nachneutralisiert. Zur Bestimmung der Farbzahl wurde ein Teil des erhaltenen Gemisches filtriert. Dazu wurde eine ungefilterte Probe im Labor mit Wasserstrahlvakuum über eine Porzellannutsche mit Filterpapier und Filterhilfsmittel filtriert. Als Filterpapier wurde eingesetzt: Machery Nagel MN 640 m; als Filterhilfsmittel: Filterperl D 14 von der "Deutsche Perlite GmbH, Kipperstraße 19, Dortmund. Das Filterhilfsmittel wurde vor der Filtration in einer Schichtstärke von ca. 2 cm in die Porzellannutsche auf das Filterpapier gegeben. Das filtrierte Estergemisch wies einen Isononanolgehalt von 200 Massen-ppm (bestimmt durch GC) und einen Wassergehalt von 0,025 Massen-% (bestimmt gemäß DIN 51 777) auf. Die Farbzahl (APHA/Hazen (bestimmt gemäß DIN ISO 6271 Teil 2)), bestimmt mit einem Farbzahlgerät der Firma Lange, betrug 10 mg Pt/1 und die Säurezahl lag bei 0,035 mg KOH/g.

### Beispiel 3 (Aufarbeitung gemäß der Erfindung)

In dem Reaktor, in dem sich das nach Beispiel 1 hergestellte Rohestergemisch befand, wurde nun der Anlagedruck von 600 hPa auf 10 hPa gesenkt und der Alkoholgehalt durch Destillation auf 1 Massen-% verringert. Dabei wurde keine Verdampfungsenergie dem Reaktor durch Dampfbeheizung zugeführt und die Temperatur wurde auf 225 °C abgesenkt. Anschließend wurde das Rohestergemisch bei 225 °C mit 1,5 kg einer 25 Massen-%igen wässrigen Natronlauge versetzt. Anschließend wurde der Ansatz bei gleichmäßig sinkender Temperatur im Temperaturbereich von 225 bis 195 °C einer Wasserdampfdestillation unterworfen (Dauer 1,5 h; Wassermenge: 32 kg pro h und to Rohester) Während der Wasserdampfdestillation wurde bei 200 °C die Säurezahl zu 0,06mg KOH/g bestimmt und dann weitere 0,4 kg einer 25 Massen-%igen, wässrigen Natronlauge zur Nachneutralisation zugegeben. Zehn Minuten nach der erfolgten Nachneutralisation wurde erneut die Säurezahl bestimmt. Sie lag unter der Spezifikationsgrenze von 0,04 mg KOH/g, sodass keine weitere Neutralisation erfolgte. Bei gleichmäßig sinkender Temperatur wurde die Wasserdampfdestillation bis zu einer Temperatur von 130 °C fortgesetzt (Dauer 2h). Zur Bestimmung der Farbzahl wurde ein Teil des Gemisches filtriert. Dazu wurde eine ungefilterte Probe im Labor mit Wasserstrahlvakuum über eine Porzellannutsche mit Filterpapier und Filterhilfsmittel filtriert. Als Filterpapier wurde eingesetzt: Machery Nagel MN 640 m; als Filterhilfsmittel: Filterperl D 14 von der "Deutsche Perlite GmbH , Kipperstraße 19, Dortmund. Das Filterhilfsmittel wurde vor der Filtration in einer Schichtstärke von ca. 2 cm in die Porzellannutsche auf das Filterpapier gegeben. Das filtrierte Estergemisch wies einen Isononanolgehalt von 60 Massen-ppm und einen Wassergehalt von 0,025 Massen-% (bestimmt gemäß DIN 51 777) auf. Die Farbzahl (APHA/Hazen, bestimmt gemäß DIN ISO 6271 (siehe Beispiel 2)) betrug 10 mg Pt/l und die Säurezahl lag bei 0,032 mg KOH/g.

Wie der Vergleich zwischen Beispiel 3 und Beispiel 2 zeigt, wird durch das erfindungsgemäße Verfahren die zur Aufarbeitung und Neutralisation des Veresterungsgemisch notwendige Natronlaugemenge verringert. Dadurch ergibt sich eine geringere Menge an Filtersalzanfall und eine geringere Filtrationszeit bzw. bei gleicher Filtrationszeit eine kleinere Filtrationseinheit.

Darüber hinaus erhöht sich die Ausbeute geringfiigig. Der Isononanolrestgehalt wird deutlich gesenkt. Zur Absenkung auf einen spezifizierten Isononanolrestgehalt (< 0,015 Massen-%) wird deutlich weniger Energie benötigt als im Vergleichsbeispiel, da zu Beginn der Aufarbeitung keine zusätzliche Wärmeenergie aufgewendet werden muss.

## Patentansprüche

1. Verfahren zur Herstellung von Carbonsäureestern durch metallkatalysierte Reaktion von Mono-, Di- oder Polycarbonsäuren oder deren Anhydriden mit Alkohol in Gegenwart eines Überschusses an Alkohol, wobei als Metallkatalysator Titankatalysatoren eingesetzt werden und der überschüssige Alkohol nach der Veresterung entfernt, der so erhaltene Rohester durch Basenzugabe neutralisiert und anschließend filtriert wird, und wobei ein Teil des überschüssigen Alkohols durch mindestens eine Wasserdampfdestillation entfernt wird,
**dadurch gekennzeichnet,**
**dass**
a) in einem ersten Schritt durch Destillation der Alkoholgehalt im Veresterungsgemisch auf einen Anteil von kleiner-gleich 5 Massen-% reduziert wird,
b) dem in Schritt a) erhaltenen Rohester eine erste Menge Base zugesetzt wird, so dass die Base, in Basenäquivalenten gerechnet, mit dem Metallatom des eingesetzten Veresterungskatalysator im molaren Verhältnis von 10 zu 1 bis 1 zu 1 steht,
c) das in Schritt b) erhaltene Gemisch einer Wasserdampfdestillation unterzogen wird und d) dass im Verlauf der Wasserdampfdestillation eine zweite Menge Base dem Gemisch zugegeben wird, die mindestens der Menge entspricht, die zur Neutralisation von Restsäure notwendig ist.

2. Verfahren nach Anspruch 1,
**dadurch gekennzeichnet,**
**dass** bei der ersten Basenzugabe in Schritt b) soviel Base zugegeben wird, dass die Base, in Basenäquivalenten gerechnet, mit dem Metallatom des eingesetzten Veresterungskatalysators im molaren Verhältnis von 6 zu 1 bis 2 zu 1 steht.

3. Verfahren nach Anspruch 1 oder 2,
**dadurch gekennzeichnet,**
**dass** die Katalysatorkonzentration zu Beginn der Veresterungsreaktion von 0,005 bis 1 Massen-% bezogen auf das Reaktionsgemisch beträgt.

4. Verfahren nach Anspruch 3,
**dadurch gekennzeichnet,**
**dass** die Katalysatorkonzentration zu Beginn der Veresterungsreaktion von 0,0075 bis 0,1 Massen-% bezogen auf das Reaktionsgemisch beträgt.

5. Verfahren nach einem der Ansprüche 1 bis 4,
**dadurch gekennzeichnet,**
**dass** als Base Natronlauge eingesetzt wird.

6. Verfahren nach einem der Ansprüche 1 bis 5,
**dadurch gekennzeichnet,**
**dass** als Mono-, Di- bzw. Polycarbonsäure Benzoesäure, Phthalsäure, 1,2-Cyclohexandicarbonsäure und/oder Trimellitsäure und/oder deren Anhydride eingesetzt werden.

7. Verfahren nach einem der Ansprüche 1 bis 6,
**dadurch gekennzeichnet,**
**dass** als Alkohol n-Butanol, Isobutanol, n-Octanol(1), n-Octanol(2), 2-Ethylhexanol, Nonanole, Decylalkohole oder Tridecanole eingesetzt werden.

8. Verfahren nach Anspruch 7,
**dadurch gekennzeichnet,**
**dass** als Alkohol Isononanol oder Isotridecanol eingesetzt wird.

9. Verfahren nach einem der Ansprüche 1 bis 8,
**dadurch gekennzeichnet,**
**dass** die Destillation im Schritt a) keine Wasserdampfdestillation ist.

10. Verfahren nach einem der Ansprüche 1 bis 9,
**dadurch gekennzeichnet,**
**dass** die Zugabe der zweiten Menge Base in Schritt d) in ein, zwei oder mehr Schritten erfolgt.

11. Verfahren nach einem der Ansprüche 1 bis 10,
**dadurch gekennzeichnet,**
**dass** zumindest eine Teilmenge der in Schritt d) zugegebenen zweiten Menge an Base zu Beginn der Wasserdampfdestillation zugegeben wird.

## Claims

1. Process for preparing esters of carboxylic acids by metal-catalyzed reaction of monobasic, dibasic or polybasic carboxylic acids or their anhydrides with alcohol in the presence of an excess of alcohol, titanium catalysts being used as metal catalyst and the excess alcohol being removed after the esterification, the resultant crude ester being neutralized by addition of base and then filtered, and at least a portion of the excess alcohol being removed by at least one steam distillation, **characterized in that**
a) in a first step, by distillation, the alcohol content in the esterification mixture is reduced to a fraction of less than or equal to 5% by mass,
b) a first amount of base is added to the crude ester obtained in step a), such that the base, calculated in base equivalents, is in a molar ratio of 10 to 1 to 1 to 1 to the metal atom of the esterification catalyst used,
c) the mixture obtained in step b) is subjected to a steam distillation and
d) in the course of the steam distillation, a second amount of base which is at least equivalent to the amount which is necessary for neutralizing residual acid is added to the mixture.

2. Process according to Claim 1, **characterized in that**, in the first addition of base in step b), sufficient base is added such that the base, calculated in base equivalents, is in the molar ratio 6 to 1 to 2 to 1 to the metal atom of the esterification catalyst used.

3. Process according to Claim 1 or 2, **characterized in that** the catalyst concentration at the start of the esterification reaction is from 0.005 to 1% by mass based on the reaction mixture.

4. Process according to Claim 3, **characterized in that** the catalyst concentration at the start of the esterification reaction is from 0.0075 to 0.1% by mass based on the reaction mixture.

5. Process according to one of Claims 1 to 4, **characterized in that** caustic soda solution is used as base.

6. Process according to one of Claims 1 to 5, **characterized in that**, benzoic acid, phthalic acid, 1,2-cyclohexanedicarboxylic acid and/or trimellitic acid and/or anhydrides thereof are used as monobasic, dibasic or polybasic carboxylic acid.

7. Process according to one of Claims 1 to 6, **characterized in that**, n-butanol, isobutanol, octan-1-ol, octan-2-ol, 2-ethylhexanol, nonanols, decyl alcohols, or tridecanols are used as alcohol.

8. Process according to Claim 7, **characterized in that**, isononanol or isotridecanol is used as alcohol.

9. Process according to one of Claims 1 to 8, **characterized in that** the distillation in step a) is not a steam distillation.

10. Process according to one of Claims 1 to 9, **characterized in that** the addition of the second amount of base in step d) is performed in one, two or more steps.

11. Process according to one of Claims 1 to 10, **characterized in that** at least a subquantity of the second amount of base added in step d) is added at the start of the steam distillation.

## Revendications

1. Procédé pour la préparation d'esters d'acides carboxyliques par réaction catalysée par des métaux d'acides monocarboxyliques, dicarboxyliques ou polycarboxyliques ou leurs anhydrides avec de l'alcool en présence d'un excès d'alcool, en utilisant comme catalyseur métallique des catalyseurs à base de titane et en éliminant l'alcool en excès après l'estérification, l'ester brut ainsi obtenu étant neutralisé par addition de base, puis filtré et une partie de l'alcool en excès étant éliminée par au moins une distillation à la vapeur d'eau, **caractérisé en ce que**
a) dans une première étape, la teneur en alcool dans le mélange d'estérification est réduite par distillation à une proportion inférieure à 5% en masse,
b) l'ester brut obtenu dans l'étape a) est additionné d'une première quantité de base, de manière telle que la base, calculée en équivalents en base, se trouve dans un rapport molaire avec l'atome métallique du catalyseur d'estérification utilisé de 10:1 à 1:1,
c) le mélange obtenu dans l'étape b) est soumis à une distillation à la vapeur d'eau et d) **en ce qu'**on ajoute, au cours de la distillation à la vapeur d'eau, une deuxième quantité de base au mélange, qui correspond au moins à la quantité nécessaire à la neutralisation de l'acide résiduel.

2. Procédé selon la revendication 1, **caractérisé en ce que** lors de la première addition de base dans l'étape b), la quantité de base ajoutée est telle que la base, calculée en équivalents en base, se trouve dans un rapport molaire avec l'atome métallique du catalyseur d'estérification utilisé de 6:1 à 2:1.

3. Procédé selon la revendication 1 ou 2, **caractérisé en ce que** la concentration en catalyseur au début de la réaction d'estérification est de 0,005 à 1% en masse par rapport au mélange réactionnel.

4. Procédé selon la revendication 3, **caractérisé en ce que** la concentration en catalyseur au début de la réaction d'estérification est de 0,0075 à 0,1% en masse par rapport au mélange réactionnel.

5. Procédé selon l'une quelconque des revendications 1 à 4, **caractérisé en ce qu'**on utilise comme base de la lessive de soude caustique.

6. Procédé selon l'une quelconque des revendications 1 à 5, **caractérisé en ce qu'**on utilise comme acide monocarboxylique, dicarboxylique ou polycarboxylique de l'acide benzoïque, phtalique, 1,2-cyclohexanedicarboxylique et/ou trimellitique et/ou leurs anhydrides.

7. Procédé selon l'une quelconque des revendications 1 à 6, **caractérisé en ce qu'**on utilise comme alcool du n-butanol, de l'isobutanol, du n-octanol (1), du n-octanol(2), du 2-éthylhexanol, des nonanols, des alcools décyliques ou des tridécanols.

8. Procédé selon la revendication 7, **caractérisé en ce qu'**on utilise comme alcool de l'isononanol ou de l'isotridécanol.

9. Procédé selon l'une quelconque des revendications 1 à 8, **caractérisé en ce que** la distillation dans l'étape a) n'est pas une distillation à la vapeur d'eau.

10. Procédé selon l'une quelconque des revendications 1 à 9, **caractérisé en ce que** l'addition de la deuxième quantité de base dans l'étape d) est réalisée en une ou deux étapes ou plus.

11. Procédé selon l'une quelconque des revendications 1 à 10, **caractérisé en ce qu'**on ajoute au moins une quantité partielle de la deuxième quantité de base ajoutée dans l'étape d) au début de la distillation à la vapeur d'eau.
